(19) Europäisches Patentamt
European Patent Office
Office européen des brevets

(11) Veröffentlichungsnummer : **0 066 753**
**B1**

(12) # EUROPÄISCHE PATENTSCHRIFT

(45) Veröffentlichungstag der Patentschrift : **30.10.85**

(51) Int. Cl.[4] : **C 07 D317/30**, C 07 D495/04 // (C07D495/04, 333:00, 235:00)

(21) Anmeldenummer : **82104448.4**

(22) Anmeldetag : **21.05.82**

(54) **Verfahren zur Herstellung von D-(+)-Biotin.**

(30) Priorität : **06.06.81 DE 3122562**

(43) Veröffentlichungstag der Anmeldung : **15.12.82 Patentblatt 82/50**

(45) Bekanntmachung des Hinweises auf die Patenterteilung : **30.10.85 Patentblatt 85/44**

(84) Benannte Vertragsstaaten : **CH DE FR GB IT LI**

(56) Entgegenhaltungen :
**LIEBIGS ANNALEN DER CHEMIE, 1980, Seiten 1972-1977, Weinheim, DE.**
**G.P. Schiemenz u.H. Engelhard, Chem. Ber. 94, (1961) 578**
**Die Akte enthält technische Angaben, die nach dem Eingang der Anmeldung eingereicht wurden und die nicht in dieser Patentschrift enthalten sind.**

(73) Patentinhaber : **Merck Patent Gesellschaft mit beschränkter Haftung**
**Frankfurter Strasse 250**
**D-6100 Darmstadt (DE)**

(72) Erfinder : **Gehlhaus, Jürgen, Dr.**
**4 Jaqueline Lane**
**Port Chester N.Y. 10572 (US)**
Erfinder : **Herz, Claus P., Dr.**
**Berghalde 20**
**D-6900 Heidelberg 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 066 753 B1

**Beschreibung**

Die Erfindung betrifft ein neues Verfahren zur stereospezifischen Synthese von D-(+)-Biotin. Stereospezifische Biotin-Synthesen, ausgehend von Zuckern geeigneter Konfiguration, sind bekannt. In Tetrahedron Letters Nr. 32, Seite 2765-2766, 1975, wird eine solche Synthese, ausgehend von D-Mannose, beschrieben. Eine verbesserte Synthese, die von D-Arabinose ausgeht und auf der Stufe des 6,9-Dihydroxy-7,8-isopropylidendioxynonansäureesters in die erstgenannte Synthese einmündet, ist in Liebig's Annalen der Chemie 1980, Seite 1972-1977, beschrieben.

Dabei wird 3,4-Isopropylidenarabinose, die in Form des cyclischen Halbacetals vorliegt, in 2-Stellung durch eine Benzoyl-Gruppe geschützt und mit einem Phosphorylid umgesetzt. Nachfolgende Hydrierung und Abspaltung der Schutzgruppe führt zum obengenannten Nonansäureester, der in bekannter Weise zu D-(+)-Biotin umgesetzt werden kann.

Auch diese verbesserte Synthese ist jedoch noch nicht befriedigend, da eine große Zahl von Reaktionsschritten notwendig ist, wodurch die Gesamtausbeute nicht sehr hoch ist.

Es bestand daher die Aufgabe, eine stereospezifische Synthese von D-(+)-Biotin zu finden, die mit wenigen Reaktionsschritten und in besserer Ausbeute zum Ziel führt.

Es wurde jetzt gefunden, daß überraschenderweise der in Liebig's Annalen der Chemie als unumgänglich angegebene Schutz der OH-Gruppe in 2-Stellung der 3,4-Isopropyliden-D-arabinose durch eine Benzoyl-Gruppe unter geeigneten Reaktionsbedingungen nicht notwendig ist, wodurch zwei Reaktionsstufen eingespart werden können und die Gesamtausbeute erhöht wird.

Gegenstand der Erfindung ist daher ein Verfahren nach dem Oberbegriff des Patentanspruchs 1, das dadurch gekennzeichnet ist, daß 3,4-Isopropyliden-D-arabinose, deren OH-Gruppe in 2-Stellung nicht geschützt ist, in einem polaren, nicht basischen Lösungsmittel zu 6,9-Dihydroxy-7,8-isopropylidendioxy-nona-2,4-diensäureester umgesetzt wird, der katalytisch hydriert und dann in an sich bekannter Weise zu D-(+)-Biotin umgesetzt wird.

Da bei der in Liebig's Annalen der Chemie beschriebenen Synthese durch Anbringen und Abspalten der Schutzgruppe ein fast 50 %iger Ausbeuteverlust eintritt, ist der Hauptvorteil des erfindungsgemäßen Verfahrens neben der Einsparung von Zeit und Gerätekapazität vor allem die Verbesserung der Gesamtausbeute an D-(+)-Biotin.

Die als Ausgangsmaterial benötigte 3,4-Isopropylidenarabinose kann zum Beispiel nach dem in Journal of the American Chemical Society 79, Seite 165, 1957, beschriebenen Verfahren hergestellt werden. Die weitere Umsetzung der Isopropylidenarabinose erfolgt nach dem folgenden Formelschema :

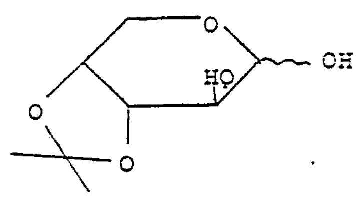 + $(C_6H_5)_3P=CH-CH=CH-COOR$

3,4-Isopropylidenarabinose + Alkoxycarbonylprop-1-en-3-yliden-triphenylphosphoran

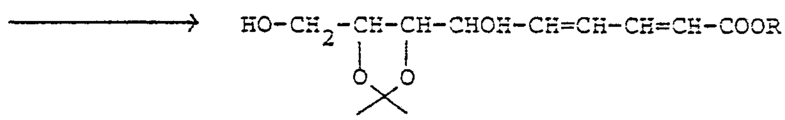

6,9-Dihydroxy-7,8-isopropylidendioxy-nona-2,4-diensäureester

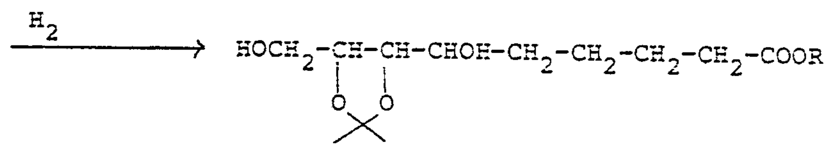


6,9-Dihydroxy-7,8-isopropylidendioxynonansäureester

bekannte
→ D-(+)-Biotin
Verfahren

Als Alkoxy-Gruppe im Phosphoran kommen insbesondere Alkyloxy-Gruppen mit etwa 1 bis 12 C-Atomen in Betracht ; bevorzugt sind Methoxy und Ethoxy.

Zur Umsetzung der Isopropylidenarabinose mit dem Phosphoran werden die beiden Komponenten in einem geeigneten Lösungsmittel gelöst und bei einer Temperatur zwischen Raumtemperatur und Siedetemperatur des Lösungsmittels, vorzugsweise bei etwa 25 bis 100 °C, für etwa 0,5 bis 24 Stunden, vorzugsweise etwa 1 bis 5 Stunden, gerührt. Im Gegensatz zu dem vorbekannten Verfahren, bei dem das Phosphoran im dreifachen molaren Überschuß eingesetzt wird, wird nach dem erfindunsgemäßen Verfahren zweckmäßig mit etwa äquimolaren Mengen gearbeitet. Dies ist ein weiterer Vorteil des erfindungsgemäßen Verfahrens.

Wichtig ist, daß als Lösungsmittel ein polares, nichtbasisches Lösungsmittel verwendet wird. Geeignet sind zum Beispiel aliphatische oder cycloaliphatische Äther, wie beispielsweise tert. Butylmethyläther, Ethylenglykoldimethyläther, Tetrahydrofuran oder Dioxan und Halogenkohlenwasserstoffe, wie biespielsweise Dichlormethan, Dichlorethan oder Chloroform.

Als entscheidend für das gute Gelingen der Reaktion wurde überraschenderweise gefunden, daß die Reaktion in neutralem bis schwach saurem Medium ablaufen muß. Offenbar wird die Reaktion durch basische Verunreinigungen, die sowohl aus der Herstellung der Isopropylidenarabinose als auch aus der Herstellung des Phosphorans stammen können, so gestört, daß keine befriedigenden Resultate erzielt werden. Werden dagegen reine Reagenzien verwendet oder basische Verunreinigungen durch Zugabe von sauren Mitteln neutralisiert, erhält man den gewünschten Nonadiensäureester in guten Ausbeuten.

Als saure Mittel sind dabei sowohl schwache Säuren, wie zum Beispiel Benzoesäure, geeignet als auch schwach oder stark saure Ionenaustauscher. Diese Mittel werden, falls notwendig, in einer solchen Menge zugegeben, daß ein leicht saures Reaktionsmedium vorliegt. In der Regel genügen katalytische Mengen von etwa 0,2 bis 1 %. In jedem Fall ist es möglich, die notwendige Menge durch wenige orientierende Vorversuche festzustellen.

Wenn die Umsetzung mit dem Phosphoran beendet ist, wird in der Regel das Lösungsmittel entfernt und aus dem verbleibenden Rückstand das gewünschte Produkt isoliert. Als geeignetes Isolierverfahren hat sich z. B. die Säulenchromatographie an Kieselgel erwiesen. Dabei gewinnt man den Nonadiensäureester als cis, trans-Isomerengemisch, in der Regel in Ausbeuten von etwa 50 bis 70 % der Theorie. Diese Nonadiensäureester sind neu und stellen ein weiteres vorteilhaftes Element der vorliegenden Erfindung dar.

Durch Hydrierung können diese Ester in die gesättigten Nonansäureester überführt werden, wobei aus dem cis, trans-Isomerengemisch eine einheitliche Verbindung entsteht. Die Hydrierung wird nach üblichen Methoden — zum Beispiel als edelmetallkatalysierte Hydrierung — bei leichtem Wasserstoffüberdruck in den dafür üblichen Lösungsmitteln durchgeführt.

Das bei der Hydrierung erhaltene Produkt ist aus Tetrahedron Letters Nr. 32, Seite 2765-2766, 1975, bekannt und kann, wie dort beschrieben, in D-(+)-Biotin überführt werden. Damit steht ein sehr vorteilhaftes Verfahren zur Herstellung von Biotin zur Verfügung.

Beispiel 1

Eine Lösung von 38,0 g (0,2 mol) 3,4-Isopropyliden-D-arabinose und 74,8 g (0,2 mol) Ethoxycarbonylprop-1-en-3-ylidentriphenylphosphoran in 500 ml Dimethoxyethan wird in Gegenwart von 2 g eines sauren Ionenaustauschers 24 Stunden bei Raumtemperatur gerührt. Nach Abtrennen des Ionenaustauschers wird das vom Lösungsmittel befreite Reaktionsgemisch an Kieselgel mit tert.-Butylmethyläther als Laufmittel chromatographiert. Man erhält 30,3 g (53 % der Theorie) 6,9-Dihydroxy-7,8-isopropylidendioxynona-2,4-diensäureethylester.

Massenspektrum (70 eV) :
m/e = 271 (3 %, $M^+—CH_3^\cdot$)
156 ( 14 %), 131 (38 %),
97 ( 23 %), 81 (24 %)
59 (100 %), 43 (35 %)

Infrarotspektrum (Film) :
3460 (OH), 2980, 2930, 2890 (C—H),
1720 (C = O), 1640 ($>C=C<$ trans),
1620 $>C=C<$ cis)

Eine Lösung von 28,6 g (0,1 mol) des erhaltenen Produkts in 200 ml Methanol wird nach Zusatz von 5 g 5 % Pd/MgO-Katalysator bei 20 °C und 1 bar Wasserstoffdruck bis zum Stillstand der Wasserstoffaufnahme hydriert. Nach Abfiltrieren des Katalysators und Entfernen des Lösungsmittels bleiben 29,0 g (100 % der Theorie) 6,9-Dihydroxy-7,8-isopropylidendioxy-nonansäureethylester.

Beispiel 2

Eine Lösung von 187 g Ethoxycarbonylprop-1-en-3-yliden-triphenylphosphoran, 95 g Isopropylidenarabinose und 2 g Benzoesäure in einem Liter Dioxan wird 3 Stunden bei 60 bis 70 °C gerührt. Der nach Abziehen des Lösungsmittels erhaltene Rückstand enthält das cis, trans-Isomerengemisch des 6,9-Dihydroxy-7,8-isopropylidendioxynona-2,4-diensäureethylester in einer Ausbeute, die 62,8 % der Theorie entspricht. Das Produkt kann, wie in Beispiel 1 beschrieben, zum gewünschten Nonansäureethylester hydriert werden.

**Patentansprüche**

1. Verfahren zur Herstellung von D-(+)-Biotin durch Umsetzung eines D-Arabinose-Derivats mit Alkoxycarbonylprop-1-en-3-yliden-triphenylphosphoran zu einem Produkt, aus dem 6,9-Dihydroxy-7,8-isopropylidendioxynonansäureester gewonnen wird und dessen Umsetzung zu D-(+)-Biotin, dadurch gekennzeichnet, daß 3,4-Isopropyliden-D-arabinose, deren OH-Gruppe in 2-Stellung nicht geschützt ist, in einem polaren, nicht basischen Lösungsmittel zu 6,9-Dihydroxy-7,8-isopropylidendioxy-nona-2,4-diensäureester umgesetzt wird, der katalytisch hydriert und dann in an sich bekannter Weise zu D-(+)-Biotin umgesetzt wird.

2. Verfahren nach Anspruch 1, dadurch gekennzeichnet, daß die Umsetzung der Isopropylidenarabinose mit dem Phosphoran in neutralem bis schwach saurem Medium durchgeführt wird.

3. Verfahren nach Anspruch 1 oder 2, dadurch gekennzeichnet, daß Isopropylidenarabinose und das Phosphoran in etwa äquimolaren Mengen eingesetzt werden.

4. Verfahren nach einem der Ansprüche 1 bis 3, dadurch gekennzeichnet, daß die Reaktion der Isopropylidenarabinose mit dem Phosphoran bei einer Temperatur von Raumtemperatur bis zum Siedepunkt des Lösungsmittels während einer Zeit von 0,5 bis 24 Stunden durchgeführt wird.

5. Verfahren nach einem der Ansprüche 1 bis 4, dadurch gekennzeichnet, daß die Reaktion der Isopropylidenarabinose mit dem Phosphoran in Gegenwart von katalytischen Mengen einer schwachen organischen Säure durchgeführt wird.

**Claims**

1. Process for the preparation of D(+)-biotin by reacting a D-arabinose derivative with alkoxycarbonylprop-1-en-3-ylidenetriphenylphosphorane to give a product from which an ester of 6,9-dihydroxy-7,8-isopropylidenedioxynonanoic acid is obtained and converting this into D(+)-biotin, characterised in that 3,4-isopropylidene-D-arabinose in which the OH group in position 2 is not protected, is converted in a polar, non-basic solvent into an ester of 6,9-dihydroxy-7,8-isopropylidene-dioxynona-2,4-dienoic acid, which is catalytically hydrogenated and then converted into D(+)-biotin in a manner know per se.

2. Process according to Claim 1, characterised in that the reaction of the isopropylidenearabinose with the phosphorane is carried out in a neutral to slightly acid medium.

3. Process according to Claim 1 or 2, characterised in that isopropylidenearabinose and the phosphorane are employed in approximately equimolar amounts.

4. Process according to one of the Claims 1 to 3, characterised in that the reaction of isopropylidenearabinose with the phosphorane is carried out at a temperature from room temperature up to the boiling point of the solvent for a period of 0.5 to 24 hours.

5. Process according to one of the Claims 1 to 4, characterised in that the reaction of the isopropylidenearabinose with the phosphorane is carried out in the presence of catalytic amounts of a weak organic acid.

**Revendications**

1. Procédé de préparation de la D-(+)-biotine par réaction d'un dérivé du D-arabinose avec un alcoxycarbonylpropa-1-ène-3-ylidène-triphénylphosphorane conduisant à un produit à partir duquel on isole l'ester 6,9-dihydroxy-7,8-isopropylidène-dioxy-nonanoïque qu'on convertit en D-(+)-biotine, caractérisé en ce que l'on convertit le 3,4-isopropylidène-D-arabinose dont le groupe OH en position 2 n'est pas protégé, dans un solvant polaire non basique, en ester 6,9-dihydroxy-7,8-isopropylidène-dioxy-nona-2,4-diénoïque qu'on soumet à hydrogénation catalytique, après quoi on convertit de manière connue en soi en la D-(+)-biotine.

2. Procédé selon la revendication 1, caractérisé en ce que la réaction entre l'isopropylidène-arabinose et le phosphorane est effectuée en milieu neutre à légèrement acide.

3. Procédé selon la revendication 1 ou 2, caractérisé en ce que l'on met en œuvre l'iso-propylidène-arabinose et le phosphorane en quantités à peu près équimoléculaires.

4. Procédé selon l'une des revendications 1 à 3, caractérisé en ce que la réaction entre l'isopropylidène-arabinose et le phosphorane est effectuée à une température comprise entre la température ambiante et le point d'ébullition du solvant dans une durée de 0,5 à 24 heures.

5. Procédé selon l'une des revendications 1 à 4, caractérisé en ce que la réaction entre l'isopropylidène-arabinose et le phosphorane est effectuée en présence de quantités catalytiques d'un acide organique faible.